(19) Europäisches Patentamt / European Patent Office / Office européen des brevets

(11) **EP 4 335 450 A1**

(12) **EUROPEAN PATENT APPLICATION**
published in accordance with Art. 153(4) EPC

(43) Date of publication:
**13.03.2024 Bulletin 2024/11**

(21) Application number: **22799102.3**

(22) Date of filing: **03.05.2022**

(51) International Patent Classification (IPC):
**A61K 38/00** (2006.01)    **A61K 38/17** (2006.01)
**A61K 39/00** (2006.01)    **A61P 35/00** (2006.01)

(52) Cooperative Patent Classification (CPC):
**A61K 38/00; A61K 38/17; A61K 39/00; A61P 35/00**

(86) International application number:
**PCT/KR2022/006352**

(87) International publication number:
**WO 2022/235059 (10.11.2022 Gazette 2022/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.05.2021 KR 20210057060**

(71) Applicant: **Cellemedy Co., Ltd
Gyeonggi-do 13605 (KR)**

(72) Inventors:
• **LEE, Jee Won
Seoul 06707 (KR)**
• **LEE, Bo Ram
Seongnam-si Gyeonggi-do 13557 (KR)**

(74) Representative: **dompatent von Kreisler Selting Werner -
Partnerschaft von Patent- und Rechtsanwälten mbB
Deichmannhaus am Dom
Bahnhofsvorplatz 1
50667 Köln (DE)**

Remarks:
The complete document including Reference Table(s) and the Sequence Listing(s) can be downloaded from the EPO website

(54) **PHARMACEUTICAL COMPOSITION FOR PREVENTION OR TREATMENT OF LUNG CANCER**

(57) The present invention relates to a protein in which a molecule capable of binding to an immune checkpoint molecule is fused, and a use thereof. The protein of the present invention has the molecule capable of binding to the immune checkpoint molecule fused to an outer surface thereof and thus may be used as an immune checkpoint inhibitor.

[FIG. 5]

**EP 4 335 450 A1**

**Description**

[Technical Field]

The present invention relates to a pharmaceutical composition for prevention or treatment of lung cancer.

[Background Art]

With the development of medical technology in modern times, incurable diseases have almost disappeared. However, unlike other diseases, cancer requires a very difficult and complex treatment. The methods currently used for cancer treatment generally include surgery, radiation therapy and chemotherapy. If the cancer develops locally without metastasizing to other sites, it can be treated through surgery to remove the cancer. However, since cancer metastasis occurs in more than 70% of cancer patients, adjuvant treatment regimens should be combined.

As one of the adjuvant treatment therapies, radiation therapy to kill cancer cells is executed using high-energy radiation. In the radiation therapy, the proliferation of cancer cells is inhibited when the cancer cells are irradiated with radiation, such that new cancer cells cannot be generated and cancer cells cannot be further cleaved. However, this method has a problem of involving side effects in that the radiation affects not only cancer cells but also normal cells.

Chemotherapy is an adjuvant therapy that uses drugs to kill cancer cells after surgery with the aim of killing invisible cancer cells. However, the chemotherapy has a problem of involving side effects such as vomiting, diarrhea, hair loss and the like.

In order to minimize such side effects, immunotherapeutic methods have recently emerged. Immunotherapy is a method of treating cancer using the patient's immune response, and can even prevent cancer. Cancer immunotherapy, like the principle of a vaccine, is a treatment method in which cancer-specific immune cells are activated by administering an antigen that causes tumor formation, and then the activated immune cells specifically attack cancer in the body. Further, by administering a cancer-specific antigen into the body even if the patient does not have cancer, the inactivated immune cells are activated as cancer-specific memory immune cells so as to specifically attack cancer cells when cancer occurs.

For cancer immunotherapy, it is important to deliver cancer-specific antigens (tumor-associated antigen (TAA); and tumor-specific antigen (TSA)) to lymph nodes where immune cells are concentrated. Among the tumor-specific antigens, neo-antigens, which are found in various tumor types such as lung cancer and kidney cancer, but mainly found in lung cancer and melanoma, are antigens newly generated by potential gene activity or DNA mutations in cancer patients. Therefore, this antigen is very important in the production of a 'customized cancer vaccine' based on individual genetic information of the patient.

However, conventional attempts to deliver only the cancer-specific antigen itself to lymph nodes have not been very effective. This is because the tumor antigen itself is rather short in length and the tumor antigen presentation efficiency for amplifying and activating tumor antigen-specific immune cells is remarkably low, and thus the tumor antigen-specific immunity induction efficiency is also significantly low (Non-Patent Document 1). Polymers are widely used as carriers of cancer-specific antigens in the body, and when cancer antigens are immobilized on the surface of polymers for delivery of cancer-specific antigens in the body, the cancer-specific antigens should be exposed to the particle surface through chemical bonding. However, there is still a limit in an aspect of uniformly exposing the cancer-specific antigen to the particle surface at a high density.

Compared to the conventional anticancer treatment methods, cancer immunotherapy has low side effects because it uses the patient's immune system, and therapeutic effects can last for a long time by forming immune memory. Further, it has an advantage of involving very little side effects since it has low influence on the normal cells due to a tumor antigen-specific recognition principle. In addition, due to the recent clinical success cases in cancer patients with recurrent or anticancer drug resistance, cancer immunotherapy is receiving explosive attention enough to be selected as Breakthrough of the year 2013 by Science.

Further, in the case of an antibody treatment neutralizing PD-1 (Programmed cell death protein 1)/PD-L1 (PD1 ligand), which is an immune checkpoint factor, clinical results have been obtained in melanoma and lung cancer with a response rate of 20-30% or more, therefore, within the next few years, cancer immunotherapy is likely to be used in clinical settings as a standard treatment for cancer. However, the immune checkpoint factors such as PD1/PD-L1 or CTLA4 specific immune activity inhibition blocking antibodies cannot fundamentally enhance the activity of immune cells themselves, and there is occurrence of side effects such as autoimmune diseases caused by the antibody's long residence time in the body and the Fc domain part of the antibody. In addition, high cost antibody treatment due to high consumption as well as high production costs due to the production and purification of the antibody in the animal cell system are present, which in turn cause disadvantages in terms of economical advantages.

[Summary of Invention]

[Problems to be Solved by Invention]

An object of the present invention is to provide a pharmaceutical composition for prevention or treatment of lung cancer, which includes a protein capable of binding to an immune checkpoint molecule.
Another object of the present invention is to provide a method for treating lung cancer using the above composition.

[Means for Solving Problems]

To achieve the above objects, the present invention provides a pharmaceutical composition for preventing or treating lung cancer, including a ferritin protein in which a molecule capable of binding to an immune checkpoint molecule is fused to an outer surface of the protein.
The protein may be a spherical protein formed by self-assembly of 24 ferritin monomers to which the molecule capable of binding to an immune checkpoint molecule is fused.
The molecule capable of binding to an immune checkpoint molecule may be fused inside at least one among α-helices of the ferritin monomer, or to at least one between adjacent α-helices of the ferritin monomer.
The molecule capable of binding to an immune checkpoint molecule may be fused to N-terminus or C-terminus of the ferritin monomer.
The molecule capable of binding to an immune checkpoint molecule may be fused inside A helix, B helix, C helix, D helix or E helix of the ferritin monomer.
The molecule capable of binding to an immune checkpoint molecule may be fused to A-B loop, B-C loop, C-D loop or D-E loop of the ferritin monomer.
The molecule capable of binding to an immune checkpoint molecule may be fused between N-terminus and A helix or between E helix and C-terminus of the ferritin monomer.
The protein may be mutated so that a binding force to a human transferrin receptor is reduced.
At least one among ferritin monomers constituting the protein may be characterized in that amino acid No. 15, 16, 23, 82, 84, 117, 120 or 124 in the sequence of SEQ ID NO: 1 is substituted with alanine, glycine, valine or leucine.
The binding force (K) of the ferritin protein to the transferrin receptor may satisfy Equation 1 below:

$$[\text{Equation } 1]$$

$$K \geq 10 \text{ nM}$$

(in Equation 1, K is [P][T]/[PT], wherein [P] represents a concentration of ferritin protein in an equilibrium state of a binding reaction between the ferritin protein and the transferrin receptor, [T] represents a concentration of the transferrin receptor in the equilibrium state, and [PT] represents a concentration of a complex of the ferritin protein and the transferrin receptor in the equilibrium state).
The transferrin receptor may be a human transferrin receptor.
The immune checkpoint molecule may be any one selected from the group consisting of Her-2/neu, VISTA, 4-1BBL, Galectin-9, Adenosine A2a receptor, CD80, CD86, ICOS, ICOSL, BTLA, OX-40L, CD155, BCL2, MYC, PP2A, BRD1, BRD2, BRD3, BRD4, BRDT, CBP, E2F1, MDM2, MDMX, PPP2CA, PPM1D, STAT3, IDH1, PD1, CTLA4, PD-L1, PD-L2, LAG3, TIM3, TIGIT, BTLA, SLAMF7, 4-1BB, OX -40, ICOS, GITR, ICAM-1, BAFFR, HVEM, LFA-1, LIGHT, NKG2C, SLAMF7, NKp80, LAIR1, 2B4, CD2, CD3, CD16, CD20, CD27, CD28, CD40L, CD48, CD52, EGFR family, AXL, CSF1R, DDR1, DDR2, EPH receptor family, FGFR family, VEGFR family, IGF1R, LTK, PDGFR family, RET, KIT, KRAS, NTRK1 and NTRK2.
The molecule capable of binding to an immune checkpoint molecule may be a ligand or a fragment thereof, a receptor or a fragment thereof, an antibody or a fragment thereof including an antigen binding region (CDR), which have a binding force to the immune checkpoint molecule.
The ferritin may be a human ferritin heavy chain.
The ferritin protein of the present invention may be present in an aqueous fraction of 40% or more in an E. coli production system.

[Advantageous effects]

The protein of the present invention has excellent binding force to immune checkpoint molecules.

The protein of the present invention enables T cells to kill cancer cells by preventing immune checkpoint molecules from inactivating T cells.

The proteins of the invention may fuse molecules that bind to a variety of immune checkpoint molecules.

The protein of the present invention is capable of binding to various immune checkpoint molecules.

The pharmaceutical composition for preventing or treating cancer of the present invention has excellent anti-cancer efficacy.

[Brief Description of Drawings]

FIG. 1 is a schematic diagram of a basic vector for preparing wild type (native) human ferritin heavy chain (huHF) and mutant human ferritin heavy chain protein.

FIG. 2 is a schematic diagram of a vector for producing huHF _Mutant1-dual and a diagram confirming the production of a ferritin protein.

FIG. 3 is a schematic diagram of a vector for producing huHF_Mutant1-h_smPD1 and a diagram confirming the production of the ferritin protein.

FIG. 4 illustrates results of evaluating the targeting ability of the ferritin protein against the mouse lung cancer cell line LLC1.

FIG. 5 illustrates results of evaluating the targeting ability of the ferritin protein against the human lung cancer cell line A549.

FIG. 6 illustrates results of evaluating the targeting ability of the ferritin protein against the mouse lung cancer cell line LLC1.

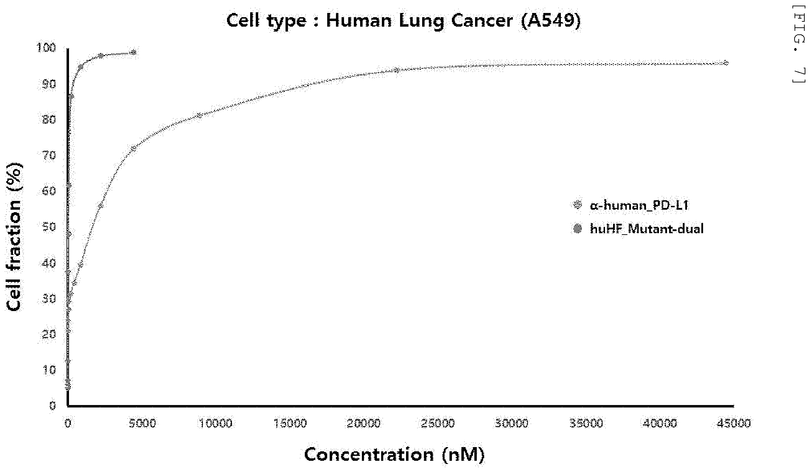

FIG. 7 illustrates results of evaluating the targeting ability of the ferritin protein against the human lung cancer cell line A549.

FIGS. 8 and 9 illustrate results of evaluating the binding force of huHF_native-h_smPD1 and huHF_Mutant1-h_smPD1 to human transferrin receptor, respectively.

FIG. 10 illustrates results of evaluating the binding force of huHF _Mutant1-dual to human transferrin receptor.

FIG. 11 illustrates results of evaluating the binding force of huHF _Mutant1-dual and huHF_Mutant2-dual to human transferrin receptor.

FIG. 12 illustrates results of evaluating the binding ability between NK cells and cancer cells according to huHF_mutant1-dual treatment.

FIGS. 13 and 14 illustrate results of evaluating the binding ability between NK cells and cancer cells according to huHF_mutant1-dual treatment.

FIG. 15 illustrates results of evaluating the cancer cell killing ability of NK cells according to huHF_mutant1-dual treatment.

FIG. 16 is a schematic diagram showing the preparation of an animal model of LLC1 lung cancer and tumor suppression experiment.

FIGS. 17 and 18 illustrate results of evaluating tumor growth inhibition according to huHF_mutant1-dual treatment.

FIGS. 19 to 21 illustrate results of evaluating avidity for the lung cancer cell line A549 of huHF_mutant1-dual.

FIG. 22 illustrates results of evaluating tumor growth inhibition in the animal model of LLC1 lung cancer when using huHF_mutant1-dual or in combination with an anticancer agent.

FIGS. 23 and 24 are schematic diagrams of a vector for producing a ferritin protein in which a foreign peptide is fused to a helix, and illustrate protein production results, respectively.

[Mode for Carrying out Invention]

The present invention relates to a pharmaceutical composition for preventing or treating lung cancer, which includes a ferritin protein in which a molecule capable of binding to an immune checkpoint molecule is fused to an outer surface of the protein

The ferritin may be ferritin derived from humans, animals and microorganisms.

Human ferritin is composed of a heavy chain (21 kDa) and a light chain (19 kDa), and exhibits the feature of forming spherical nanoparticles through self-assembly ability of monomers constituting the ferritin. The ferritin may form a self-assembly having a spherical three-dimensional structure by gathering 24 monomers.

For the human ferritin, an outer diameter is about 12 nm and an inner diameter is about 8 nm. The structure of a ferritin monomer is a form in which five α-helix structures, namely, A helix, B helix, C helix, D helix and E helix are sequentially connected, and may include an atypical polypeptide portion that links polypeptides having each α-helix structure called a loop.

The loop is a region that is not structurally broken even when a peptide or a small protein antigen is inserted into the ferritin. Herein, the peptide is fused using cloning, such that a peptide-ferritin fusion protein monomer, in which a peptide such as an epitope is located in the ferritin monomer, may be prepared. The loop linking A helix and B helix is called A-B loop, the loop linking B helix and C helix is called B-C loop, the loop linking C helix and D helix is called C-D loop, and the loop linking D helix and E helix is called D-E loop.

Information on ferritin is known from the NCBI (GenBank Accession No. NM_000146, NM_002032 or the like).

The ferritin may be a ferritin heavy chain, and specifically, a human ferritin heavy chain. The human ferritin heavy chain may be a protein represented by the amino acid sequence of SEQ ID NO: 1 derived from humans, and in the present specification, the ferritin may be used interchangeably with 'human ferritin heavy chain' or 'huHF'.

The ferritin protein may be a ferritin monomer. Accordingly, it may be a ferritin heavy chain monomer or a human ferritin heavy chain monomer.

Ferritin forms an organized structure or pattern by self-assembly of several monomers thereof. The ferritin protein of the present invention is nano-scaled particles. For example, 24 ferritin monomers fused with a molecule capable of binding to an immune checkpoint molecule may be self-assembled to form a ferritin protein.

The ferritin protein may have a spherical shape. In the case of the spherical shape, for example, the particle diameter may be 8 to 50 nm. More specifically, it may be 8 to 50 nm, 8 to 45 nm, 8 to 40 nm, 8 to 35 nm, 8 to 30 nm, 8 to 25 nm, 8 to 20 nm, or 8 to 15 nm.

The ferritin protein is resulted from fusion of a molecule capable of binding to an immune checkpoint molecule on an outer surface thereof. As long as the molecule capable of binding to the immune checkpoint molecule is fused to the outer surface of the ferritin protein, it is not necessary that the molecule capable of binding to an immune checkpoint molecule is fused to all ferritin monomers constituting the ferritin protein.

For example, when 24 ferritin monomers fused with molecules capable of binding to an immune checkpoint molecule are self-assembled to form a ferritin protein, it may be sufficient that a molecule capable of binding to an immune checkpoint molecule is fused to at least one ferritin monomer among the 24 ferritin monomers. In order to increase the density of molecules capable of binding to immune checkpoint molecules on the outer surface of the ferritin protein, all 24 ferritin monomers can be fused to the molecules capable of binding to immune checkpoint molecules.

One or two or more molecules capable of binding to an immune checkpoint molecule may be fused to each ferritin monomer constituting the ferritin protein. Each ferritin monomer constituting the ferritin protein may be fused with molecules capable of binding to different immune checkpoint molecules. Each ferritin monomer constituting the ferritin protein may be fused with different molecules capable of binding to the same immune checkpoint molecule.

Immune checkpoint molecules may bind to T cells and inactivate the same. Such immune checkpoint molecules may include, for example, Her-2/neu, VISTA, 4-1BBL, Galectin-9, Adenosine A2a receptor, CD80, CD86, ICOS, ICOSL, BTLA, OX-40L, CD155, BCL2, MYC, PP2A, BRD1, BRD2, BRD3, BRD4, BRDT, CBP, E2F1, MDM2, MDMX, PPP2CA, PPM1D, STAT3, IDH1, PD1, CTLA4, PD-L1, PD-L2, LAG3, TIM3, TIGIT, BTLA, SLAMF7, 4-1BB, OX-40, ICOS, GITR, ICAM-1, BAFFR, HVEM, LFA-1, LIGHT, NKG2C, SLAMF7, NKp80, LAIR1, 2B4, CD2, CD3, CD16, CD20, CD27, CD28, CD40L, CD48, CD52, EGFR family, AXL, CSF1R, DDR1, DDR2, EPH receptor family, FGFR family, VEGFR family, IGF1R, LTK, PDGFR family, RET, KIT, KRAS, NTRK1, NTRK2 and the like.

The molecule capable of binding to the immune checkpoint molecule may be a ligand or a fragment thereof, a receptor or a fragment thereof, an antibody or a fragment thereof including an antigen binding site (CDR), which have a binding force to the immune checkpoint molecule.

Molecules capable of binding to immune checkpoint molecules may be fused to any portion of a ferritin monomer as long as they can be exposed to the outer surface of the ferritin protein. The molecules capable of binding to immune checkpoint molecules are fused at a position that does not interfere with self-assembly of ferritin monomers.

The molecules capable of binding to immune checkpoint molecules can be fused inside the ferritin monomer to change the structure of the ferritin protein. Among the respective components of the ferritin monomer, the internally indented part may protrude to an outside by fusion of the same with the molecule capable of binding to the immune checkpoint molecule. On the other hand, the portion protruding to the outside among the respective components of the ferritin monomer may be internally indented by fusion of the same with the molecule capable of binding to the immune checkpoint molecule.

The molecule capable of binding to the immune checkpoint molecule is preferably fused to a position where the ferritin protein reduces the binding force to the human transferrin receptor or interferes with the binding to the human transferrin receptor.

The molecule capable of binding to an immune checkpoint molecule is not limited to a specific range in terms of the structure, molecular weight, and amino acid length thereof.

The molecule capable of binding to an immune checkpoint molecule may be, for example, a ligand, antibody, or fragment thereof whose amino acid length is 25aa or less. More specifically, the amino acid length may be 25aa or less, 24aa or less, 23aa or less, 22aa or less, 21aa or less, 20aa or less, 19aa or less, 18aa or less, 17aa or less, 16aa or less, 15aa or less, 14aa or less, 13aa or less, 12aa or less, 11aa or less or less, 10aa or less, 9aa or less, 8aa or less, 7aa or less, 6aa or less, 5aa or less. Further, for example, the length of amino acid may be a 3aa or more, 4aa or more, 5aa or more, 6aa or more, 7aa or more, 8aa or more, 9aa or more, 10aa or more.

A fusion site of the molecule capable of binding to the immune checkpoint molecule is not limited to a specific position, for example, the fusion may be performed inside the $\alpha$-helix (A helix, B helix, C helix, D helix, or E helix), between adjacent $\alpha$-helices, N-terminus, C-terminus, A-B loop, B-C loop, C-D loop, D-E loop, between N-terminus and A helix, between E helix and C-terminus, inside helix, etc. of the ferritin monomer.

Further, the protein of the present invention may further include a foreign peptide fused inside the $\alpha$-helix (A helix, B helix, C helix, D helix, or E helix), between adjacent $\alpha$-helices, N-terminus, C-terminus, A-B loop, B-C loop, C-D loop, D-E loop, between N-terminus and A helix, between E helix and C-terminus, inside helix, etc. of the ferritin monomer. The foreign peptide may be a pharmacologically active peptide.

Since the ferritin protein is designed to bind to an immune checkpoint molecule, it is preferable that it does not bind well to a transferrin receptor. For example, the ferritin protein satisfies the following Equation 1 for the transferrin receptor:

$$[\text{Equation 1}]$$

$$K \geq 10 \text{ nM}$$

(in Equation 1, K is [P][T]/[PT], wherein [P] represents a concentration of ferritin protein in an equilibrium state of a binding reaction between the ferritin protein and the transferrin receptor, [T] represents a concentration of transferrin receptor in the equilibrium state, and [PT] represents a concentration of a complex of the ferritin protein and the transferrin receptor in the equilibrium state).

The binding force may be, for example, a binding force to the human transferrin receptor.

K value of Equation 1 is 10 nM or more, 20 nM or more, 30 nM or more, 40 nM or more, 50 nM or more, 60 nM or more, 70 nM or more, 80 nM or more, 90 nM or more, 100 nM or more, 110 nM or more, 120 nM or more, 125 nM or more, more than 125 nM, 150 nM or more, 200 nM or more, 210 nM or more, 220 nM or more, 230 nM or more, 240 nM or more, 250 nM or more, 260 nM or more, 270 nM or more, 280 nM or more, 290 nM or more, 300 nM or more, 350 nM or more, 400 nM or more, 450 nM or more, 500 nM or more, 550 nM or more, 600 nM or more, 700 nM or more, 800 nM or more, 900 nM or more, or 1000 nM or more. The higher the value of K in Equation 1, the lower the binding force to the transferrin receptor. The upper limit may be, for example, 100,000 nM, 90,000 nM, 80,000 nM, 70,000 nM, 60,000 nM, 50,000 nM, 10,000 nM, 9,000 nM, 8,000 nM, 7,000 nM, 6,000 nM, 5,000 nM, or the like, but it is not limited thereto.

The binding force (K) to the transferrin receptor is measured in the equilibrium state of the binding reaction between the ferritin protein (A) of the present invention and the transferrin receptor. The concentration of the ferritin protein ([P]), the concentration of the transferrin receptor ([T]), and the concentration of the complex of the protein of the present invention and the transferrin receptor ([PT]) in the equilibrium state can be measured by various known methods.

The ferritin protein may fuse the molecule capable of binding to an immune checkpoint molecule to a site involved in binding to the transferrin receptor in order to decrease the binding force to the transferrin receptor. For example, this protein may fuse the molecule capable of binding to an immune checkpoint molecule to be located at the B-C loop and the A helix portion of the ferritin protein of the present invention.

The ferritin protein may be mutated so that binding to the human transferrin receptor is reduced. For example, amino acid No. 15, 16, 23, 82, 84, 117, 120 or 124 in the sequence of SEQ ID NO: 1 may be substituted with alanine, glycine, valine or leucine.

The ferritin protein may be produced in a microorganism expressing a sequence encoding the corresponding protein.

Microorganisms known in the art may be used without limitation thereof. For example, it may be *E. coli*, and specifically BL21 (DE3), but it is not limited thereto.

In the case of producing a protein by a microbial system, it is easy to perform separation/purification only when the obtained protein is present in a dissolved state in the cytoplasm. In many cases, the prepared protein is present in an aggregated state as an inclusion body or the like. The ferritin protein of the present invention has a high rate of lysis in the cytoplasm in a microbial production system. Therefore, it is easy to perform separation/purification and utilize the same.

The ferritin protein may be prepared, for example, in an *E. coli* system for producing the same in a state in which the water-soluble fraction ratio of the total protein is 40% or more. Specifically, the fraction ratio may be 40% or more, 50% or more, 60% or more, 70% or more, 80% or more, 90% or more, or 95% or more. The upper limit thereof may be, for example, 100%, 99%, 98%, 97%, 96% or the like.

The pharmaceutical composition of the present invention may include a pharmaceutically acceptable carrier. As used herein, the term "pharmaceutically acceptable carrier" refers to a carrier or diluent that does not inhibit the biological activity and properties of the administered component without significantly stimulating the organism. The pharmaceutically acceptable carrier in the present invention may be one component or a mixture of one or more components among saline, sterile water, Ringer's solution, buffered saline, dextrose solution, maltodextrin solution, glycerol and ethanol. Further, as necessary, other conventional additives such as antioxidants, buffers and bacteriostatic agents may be added to formulate an injection suitable for injection into tissues or organs. Furthermore, it may be formulated as an isotonic sterile solution, or in some cases, a dry preparation (especially a freeze-dried preparation) that can be an injectable solution by adding sterile water or physiological saline. In addition, a target organ-specific antibody or other ligand may be used in combination with the carrier so as to act specifically on the target organ.

Further, the composition of the present invention may further include a filler, an excipient, a disintegrant, a binder or a lubricant. Furthermore, the composition of the present invention may be formulated using methods known in the art to provide rapid, sustained or delayed release of the active ingredient after administration to a mammal.

In one embodiment, the pharmaceutical composition may be an injection formulation, and may be administered intravenously, but it is not limited thereto.

As used herein, the term "effective amount" refers to an amount necessary to delay the onset or progression of a specific disease to be treated or to promote it entirely.

In the present invention, the composition may be administered in a pharmaceutically effective amount. It is apparent to those skilled in the art that a suitable total daily amount of the pharmaceutical composition may be determined by a treating physician within the scope of sound medical judgment.

For the purposes of the present invention, a specific pharmaceutically effective amount for a specific patient is preferably applied differently depending on various factors including: type and extent of the response to be achieved; whether or not other agents are used if necessary; specific composition; patient's age, weight general health status, sex, diet; administration time; administration route; secretion rate of the composition; treatment period; drug used together or concurrently with the specific composition, as well as similar factors well known in the pharmaceutical field.

In the present invention, the pharmaceutical composition may be accompanied by an instruction associated with packaging in a form instructed by a government agency in charge of the manufacture, use and sale of drugs if necessary. The instruction indicates the approval of a private interest organization in regard to the form of a composition or administration to human or animals, and may be, for example, a label approved by the US Food and Drug Administration for the prescription of the drugs.

The pharmaceutical composition of the present invention may further include an anticancer agent, or may be co-administered with an anticancer agent.

As an anticancer agent, any substance having anticancer activity may be used without limitation thereof, and known anticancer agents may also be used.

For example, the anticancer agent may be an anticancer chemotherapeutic agent, a targeted anticancer agent, or an anticancer immunotherapeutic agent.

The anticancer chemotherapeutic agent may be an alkylating agent, a microtubule inhibitor, an antimetbolite, or a topoisomerase inhibitor, but it is not limited thereto.

The alkylating agent may be mechlorethamine, cyclophosphamide, ifosfamide, melphalan, chlorambucil, thiotepa, altretamine, procarbazine, busulfan, streptozocin, carmustine, iomustine, dacarbazine, cisplatin, carboplatin or oxaliplatin, etc., but it is not limited thereto. The microtubule inhibitor may be docetaxel, vinblastine, oncovin or vinorelbine, etc., but it is not limited thereto. The antimetbolite may be fluorouracil, capecitabine, cytarabine, gemcitabine, fludarabine, methotrexate, pemetrexed or mercaptopurine, etc., but it is not limited thereto. The topoisomerase inhibitor may be hycamtin, camptosar, vepesid, taxol, bleomycin, adriamycin or cerubidine, etc., but it is not limited thereto.

The targeted anticancer agent may be trastuzumab, pertuzumab, panitumumab, cetuximab, bevacizumab, ramucirumab, aflibercept, rituximab, obinutuzumab, daratumumab, denosumab, ibrutinib, dasatinib, nilotinib, imatinib, bosutinib, osimertinib, erlotinib, gefitinib, nintedanib, sunitinib, sorafenib, cabozantinib, lenvatinib, regorafenib, axitinib, pazopanib, cabozantinib, trametinib, dabrafenib, abemaciclib, palbociclib, lenalidomide, ruxolitinib, alectinib, crizotinib, olaparib or venetoclax, etc., but it is not limited thereto.

The immuno-cancer agent may be an immune checkpoint inhibitor, an immune cell therapeutic agent (CAR-T), an antibody drug conjugate (ADC), a dual antibody, an anti-cancer virus or an anti-cancer vaccine, but it is not limited thereto.

The checkpoint inhibitor may be PD-1 antibody, PD-L1 antibody, CTLA-4 antibody, TIM3 antibody or LAG3 antibody. The PD-1 antibody may be pembrolizumab, nivolumab or cemiplimab, and the PD-L1 antibody may be atezolizumab, avelumab or durvalumab, but they are not limited thereto. Further, the CTLA-4 antibody may be ipilimumab or tremelimumab, the TIM3 antibody may be MBG452, and the LAG3 antibody may be BMS-986016 or LAG525, but they are not limited thereto. Further, the immune cell therapeutic agent may be tisagenlecleucel or axicabtagene ciloleucel, but it is not limited thereto. The ADC may be gemtuzumab-ozogamicin, brentuximab vedotin, trastuzumab emtansine, inotuzumab ozogamicin or eribulin mesylate, but it is not limited thereto. The double antibody may be blinatumomab, the anticancer virus may be talimogene laherparepvec, and the anticancer vaccine may be sipuleucel-T, but they are not limited thereto.

The anticancer agent may be an anticancer agent for lung cancer.

The pharmaceutical composition of the present invention may be administered as an individual therapeutic agent or in combination with other therapeutic agents, and may be administered simultaneously, separately or sequentially with the conventional anticancer agents. In addition, it may be administered in single or multiple doses. Taking all of the above factors into consideration, it is important to administer the pharmaceutical composition in an amount that can achieve maximum effects with a minimum amount without side effects, which may be easily determined by those skilled in the art.

The term "simultaneously" means occurring or being performed simultaneously, unless otherwise specified, and the term "separately" means keeping separate from each other, unless otherwise specified. Further, the term "sequentially" means, unless otherwise specified, being characterized by a regular sequence or order, and the sequential dosing regimen may include administering a composition including an anticancer agent before, simultaneously with, or after administration of another composition including an extract, however, both compositions will be administered in a regular sequence or order. The sequential administration refers to the temporally separate administration of the compositions described herein.

The present invention provides a method for treatment of lung cancer using the above ferritin protein. All of the above descriptions regarding the ferritin protein will be applied as they are to the ferritin protein as an active ingredient in the cancer treatment method of the present application.

The treatment method of the present invention may include administering the ferritin protein of the present invention to a subject suffering from lung cancer.

The individual suffering from lung cancer may be an animal suffering from lung cancer, specifically, a mammal suffering from lung cancer, and more specifically, a human suffering from lung cancer.

The protein may be administered in a therapeutically effective amount.

In the present invention, the term "administration" means introducing the composition of the present invention to a patient by any suitable method, and the administration route for the composition of the present invention may include various administration routes, either oral or parenteral, as long as it can reach the target tissue. It may include intraperitoneal administration, intravenous administration, intramuscular administration, subcutaneous administration, intradermal administration, oral administration, topical administration, intranasal administration, intrapulmonary administration or rectal administration, but it is not limited thereto.

The method of the present invention may further include administering an anticancer agent to the subject.

As the anticancer agent, any substance having anticancer activity may be used without limitation thereof, for example, the anticancer agent exemplified above may be used.

The anticancer agent may be administered simultaneously, separately or sequentially with the ferritin protein.

Hereinafter, the present invention will be described in detail with reference to the following examples.

**EXAMPLE**

**1. Construction of expression vector for production of protein**

huHF is spherical protein nanoparticles (12 nm) composed of 24 monomers, each of which is composed of a total of 5 $\alpha$-helices. The present inventors induce mutation in the amino acid sequence that binds to a transferrin receptor of the huHF monomer through gene cloning, such that huHF_Mutant1 (Q15A, D16A, R23A, F82A, Q84A), huHF_Mutant2 (Q15A, D16A, R23A, F82A, Q84A, E117A, K120A, D124A) were obtained as shown in Table 1 below. The antibody CDR3 peptide and domain were inserted into a selected position among the loop between $\alpha$-helices of huHF_Mutant1 and 2 (BC loop in huHF 5T to 176G based on the PDB 3AJO sequence; 92D/93W) and the C-terminus through gene cloning, so as to ensure the delivery system.

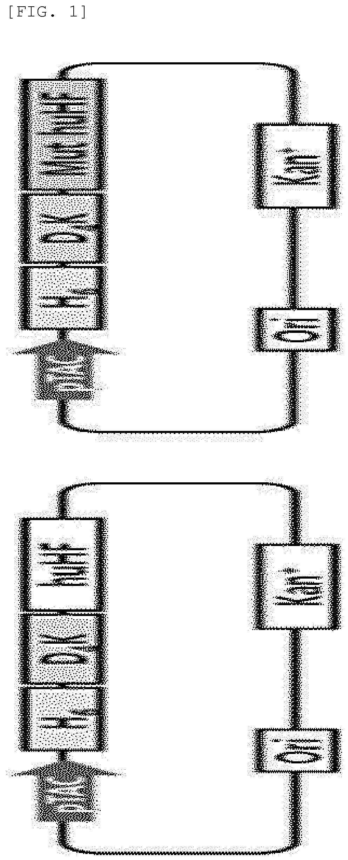

The sequence of Table 2 below was used, and PCR was performed according to the vector schematic diagram of FIGS. 1 to 3, and Table 3, thereby preparing huHF_Mutant1_1_dual (BC loops, C-terminus), huHF_Mutant2-dual (BC loops, C-terminus) and huHF_Mutant1-h_smPD1 (C-terminus). All the constructed plasmid expression vectors were purified on an agarose gel, and then the sequence was identified through complete DNA sequencing.

[TABLE 1]

| Designation | SEQ ID NO. |
|---|---|
| huHF_Native | 1 |
| huHF-Mutant1 | 2 |
| huHF-Mutant2 | 3 |

[TABLE 2]

| Designation | SEQ ID NO. |
|---|---|
| huHF-αPD-L1 | 4 |
| huHF αTIGIT | 5 |
| huHF-h_smPD1 | 6 |
| Linker1 | 7 |
| Linker2 | 8 |
| Linker3 | 9 |

[TABLE 3]

| Protein | Expression vector |
|---|---|
| huHF_Native | NH2-NdeI-huHF-αPD-L1 HCDR3-HindIII-COOH |
| huHF_Mutant1-dual | BC(92D/93W): NH2-NdeI-huHF_Mutant1-[αPD-L1 HCDR3]- huHF_Mutant1- αTIGIT HCDR3-HindIII-COOH |
| huHF_Mutant2-dual | BC(92D/93W): NH2-NdeI-huHF_Mutant2-[αPD-L1 HCDR3]-huHF- αTIGIT HCDR3-HindIII-COOH |
| huHF_Mutant1-h_smPD1 | NH2-NdeI-huHF_Mutant1-Linker2-h_smPD1-HindIII-COOH |

### 2. Protein biosynthesis

*E. coli* strain BL21 [(fhuA2 [lon] ompT gal [dcm] ΔhsdS)] was transformed with the expression vector prepared above, respectively, and a kanamycin-resistant transformant was selected. Transformed *E. coli* was cultured in a flask (250 mL Erlenmeyer flask, 37 °C, 150 rpm) including 50 mL of Luria-Bertani (LB) medium (containing 100 mg L-1 kanamycin). When the medium turbidity (O.D 600) reached about 0.5-0.7, Isopropyl-β-Dthiogalactopyanosid (IPTG) (1.0 mM) was injected to induce expression of the recombinant gene.

After culturing at 20 °C for 16 to 18 hours, the cultured E. *coli* was centrifuged at 4,500 rpm for 10 minutes to recover the cell precipitate, followed by suspending the precipitate in 5 ml of a disruption solution (10 mM Tris-HCl buffer, pH 7.5, 10 mM EDTA), and then crushing the same using an ultrasonic crusher (Branson Ultrasonics Corp., Danbury, CT, USA). After crushing, centrifugation was performed at 13,000 rpm for 10 minutes to separate the supernatant and insoluble aggregates. The separated supernatant was used for subsequent experiments.

### 3. Purification of protein

The supernatant obtained in Example 2 above was purified through a three-step process. First, 1) Ni$^{2+}$-NTA affinity chromatography using the binding of histidine and nickel fused to the recombinant protein was conducted, 2) the recombinant protein was concentrated and a fluorescent material was adhered through buffer exchange, and 3) finally, in order to separate only the self-assembled protein nanoparticles to which the fluorescent material is adhered, sucrose gradient ultracentrifugation was performed. Details of each step are as follows.

1) Ni $^{2+}$-NTA affinity chromatography

In order to purify a recombinant protein, *E. coli* cultured in the same manner as specified above was recovered, cell pellets thereof were resuspended in 5 mL lysis buffer (pH 8.0, 50 mM sodium phosphate, 300 mM NaCl, 20 mM imidazole), and cells were crushed using an ultrasonicator. The lysed cell solution was centrifuged at 13,000 rpm for 10 minutes to separate only the supernatant, and then each recombinant protein was separated using a Ni$^{2+}$-NTA column (Qiagen, Hilden, Germany) (washing buffer: pH 8.0, 50 mM sodium phosphate, 300 mM NaCl, 80 mM imidazole / elution buffer: pH 8.0, 50 mM sodium phosphate, 300 mM NaCl, 200 mM imidazole).

2) Concentration, buffer exchange, and fluorescent material adhesion processes

For FACS, huHF_Native, huHF_Mutant1-h_smPD1 and huHF_Mutant1-dual particles were subjected to Ni$^{2+}$-NTA affinity chromatography, and 3 ml of the eluted recombinant protein was placed in an ultracentrifugal filter (Amicon Ultra 100K, Millipore, Billerica, MA) and centrifugation was conducted at 5,000 g until l ml of the solution remained on a column. Thereafter, in order to adhere a fluorescent material FITC (fluorescein isothiocyanate), the protein particles were buffer exchanged with sodium bicarbonate (0.1 M, pH 8.5) buffer, followed by adhering the fluorescent material at room temperature for 12 hours.

3) Sucrose gradient ultrafast centrifugation

After adding sucrose by concentration to PBS (2.7 mM KCl, 137 mM NaCl, 2 mM $KH_2PO_4$, 10 mM $Na_2HPO_4$, pH 7.4) buffer, thus to prepare each of 40%, 35%, 30%, 25% and 20% sucrose containing solutions, 2 ml of each sucrose solution at the concentration (40-20%) was placed in an ultracentrifugation tube (ultraclear 13.2 ml tube, Beckman) in sequential order from the highest concentration. Then, after filling 1 ml of the recombinant protein solution present in the prepared self-assembly buffer, ultrafast centrifugation was performed at 35,000 rpm and 4 °C for 16 hours (Ultracentrifuge L-90k, Beckman). After centrifugation, the upper layer (20-25% sucrose solution part) was carefully replaced with PBS buffer using a pipette and an ultracentrifugal filter as specified in the above item 2).

## 4. Identification of water-soluble fractions of proteins

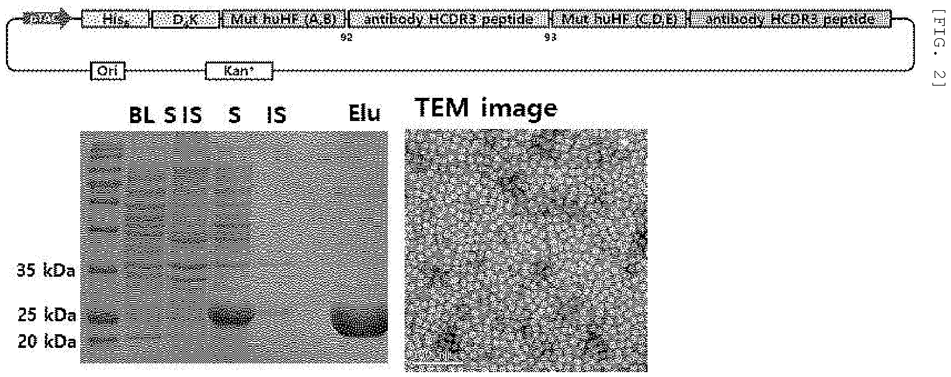

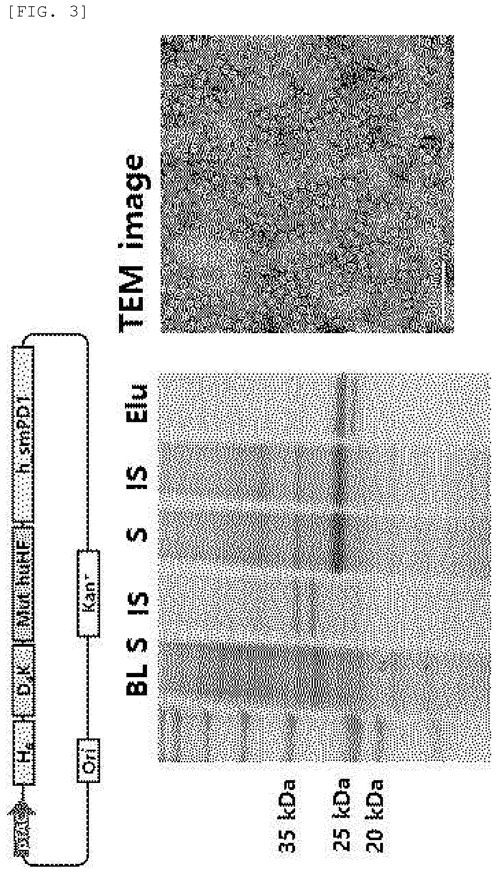

BL21 competent cells were transformed with various expression vectors based on pCM (Tac promoter). A single colony was inoculated to LB liquid medium (50 mL) supplemented with 100 mg/L of kanamycin, and cultured at 37 °C and 130 rpm in a shaking incubator. When the turbidity/optical density at 600 nm reached 0.5, the expression of a target protein was induced through administration of 1 mM IPTG. After 12-16 hours of incubation at 20 °C, the cells in the culture medium were spun-down through centrifugation (13,000 rpm, 10 minutes), and cell pellets were collected and resuspended in 10 mM Tris-Hcl (pH 7.4) buffer. The resuspended cells were ruptured using a Branson Sonifier (Branson Ultrasonics Corp., Danbury, CT). After sonication, the supernatant containing soluble protein and aggregates containing insoluble protein were separated by centrifugation (13,000 rpm, 10 min). The separated soluble and insoluble protein fractions were identified through SDS-PAGE analysis (FIGS. 2 and 3).

## 5. Verification of protein assembly

For structural analysis of the purified recombinant protein nanoparticles of each protein prepared in Example 3, the recombinant protein was photographed by a transmission electron microscope (TEM). First, unstained and purified protein samples were placed on carbon-coated copper electron microscope grids and then natural dried. To obtain stained images of the proteins, electron microscope grids containing naturally dried samples were incubated with 2% (w/v) aqueous uranyl acetate solution at room temperature for 10 minutes, and washed 3-4 times with distilled water. As a result of observing the protein image using a Philips Technai 120 kV electron microscope, it was confirmed that each particle forms spherical nanoparticles.

## 6. Verification of lung cancer cell targeting ability of the prepared proteins

huHF_Native, huHF_Mutant1-h_smPD1, and huHF_Mutant1-dual proteins prepared in Example 3, to which the fluorescent material was adhered, were subjected to comparison of lung cancer cell targeting efficiencies.

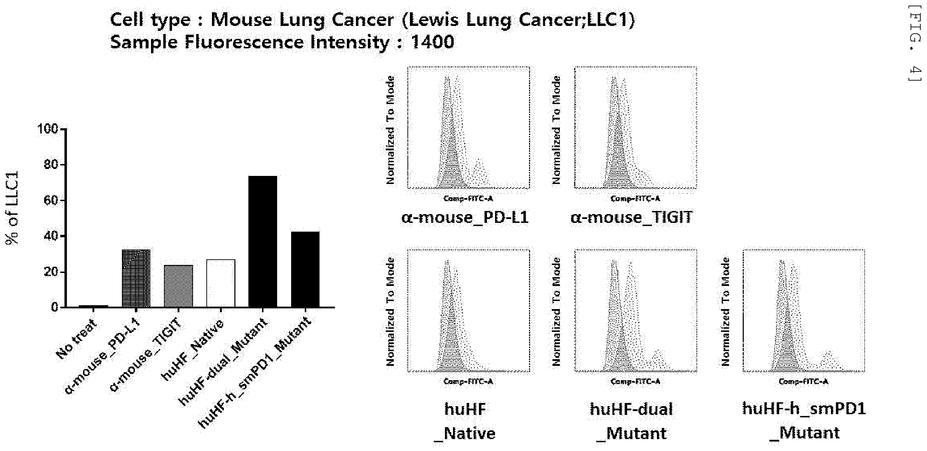

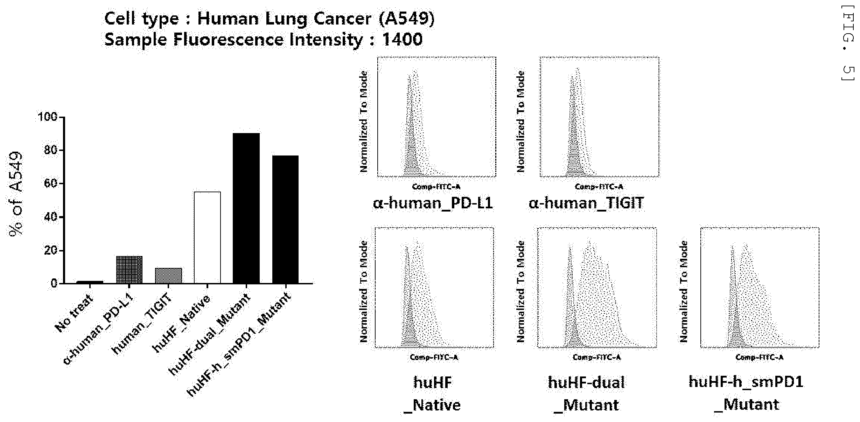

In order to compare the targeting efficiency of the huHF_Native, huHF_Mutant1-h_smPD1 and huHF_Mutant1-dual proteins prepared in Example 3 against LLC1 mouse lung cancer and A549 human lung cancer, LLC1 mouse lung cancer and A549 human lung cancer cells were reacted with the protein at a concentration having a fluorescence intensity of 1400 for 2 hours, followed by comparing fluorescence signals thus to determine lung cancer cell targeting efficiency (FIGS. 4 and 5). As a result, it was confirmed that significantly higher targeting efficiency is exhibited compared to the antibody.

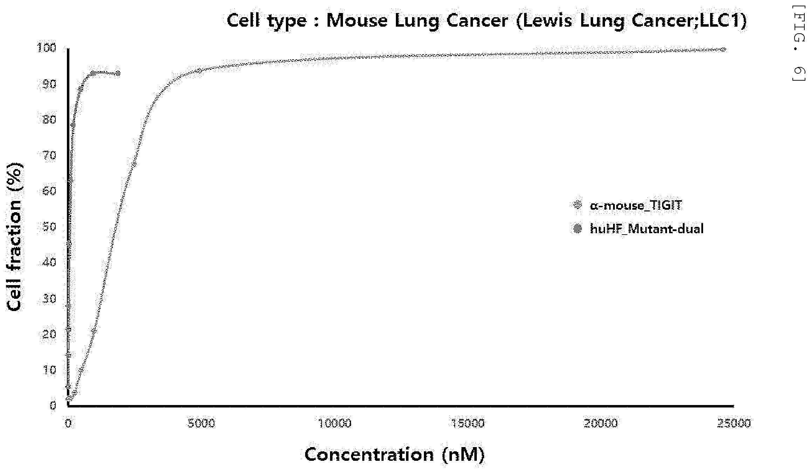

In order to compare the targeting efficiency of the huHF_Native, huHF_Mutant1-h_smPD1, and huHF_Mutant1-dual proteins prepared in Example 3 against LLC1 mouse lung cancer and A549 human lung cancer, LLC1 mouse lung cancer and A549 human lung cancer cells were reacted with the protein at a predetermined concentration section for 2 hours, then, reacted with a fluorescent antibody binding to His tag of the protein for 1 hour, followed by comparing fluorescence signals thus to determine the targeting efficiency (FIGS. 5 and 6). The concentration of 50% cell fraction may be considered as Kd, and it was confirmed that the protein exhibited 50-fold or more targeting ability compared to the antibody (Table 4).

[TABLE 4]

| Division | | Target ability |
|---|---|---|
| Antibody | Human | 889-2223 |
| | Mouse | 985-2463 |
| huHF_Mutant1-dual | Human | 44-89 |
| | Mouse | 46-93 |

## 7. Measurement of protein binding to transferrin receptor

The native form of the proteins prepared in the above examples and the binding force of the mutant to human transferrin receptor were compared.

First, human transferrin receptor-Fc tag standard was attached to a high-binding 96 well plate by 100 μl at 2 μg/ml overnight at 4 °C.

Then, it was washed 3 times with 200 ul of PBST (PBS+Tween0.05%). SuperBlcok™ (PBS) Blocking Buffer (thermo cat#37515) was subjected to a blocking process by 100 μl at room temperature for 1 hour.

Then, it was washed 3 times with 200 ul of PBST (PBS+Tween0.05%). The proteins prepared in the above examples were treated overnight at 4 °C by 100 μl for each concentration section.

Then, it was washed 3 times with 200 ul of PBST (PBS+Tween0.05%). An antibody (mouse anti-His tag antibody, Hisprobe) that specifically binds to the hexa-His tag was diluted 1/1000 in PBST, and the diluted solution was treated by 100 μl at room temperature for 1 hour.

Subsequently, it was washed 3 times with 200 ul of PBST (PBS+Tween0.05%). An antibody that specifically binds to a mouse antibody (goat anti-mouse igG antibody-HRP) was diluted 1/1000 in PBST, and the diluted solution was treated by 100 μl at room temperature for 1 hour.

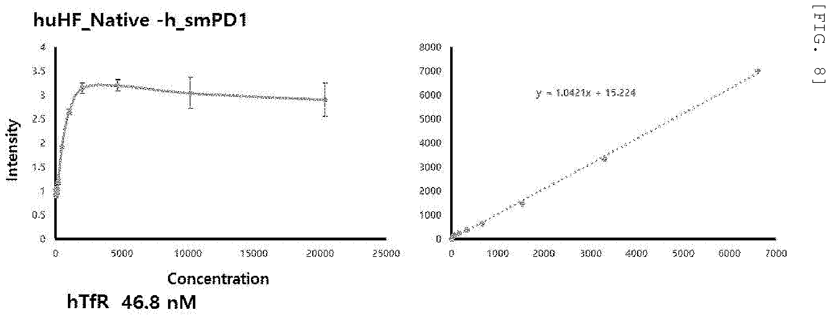

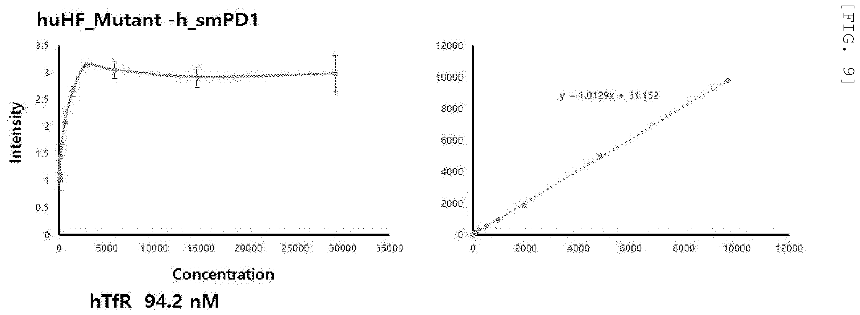

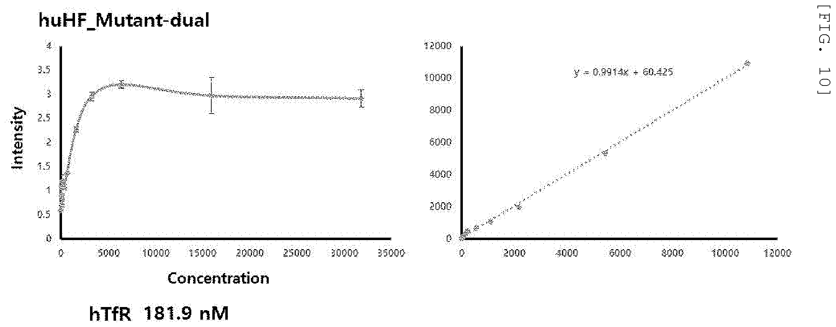

Then, it was washed 3 times with 200 ul of PBST (PBS+Tween0.05%). TMB solution expressing coloration by reacting with HRP was treated by 100 μl at room temperature for 15 minutes. After 50 μl of 1M $H_2SO_4$ solution was treated, optical density was measured with TECAN at a wavelength of 450 nm. This was calculated using the Langmuir equation (Table 5, FIGS. 8 to 10).

[TABLE 5]

| Division | | Binding force |
|---|---|---|
| huHF-h_smPD1 | Native | 46.8 |
| | Mutant | 94.2 |
| huHF-mutant1-dual | | 181.9 |

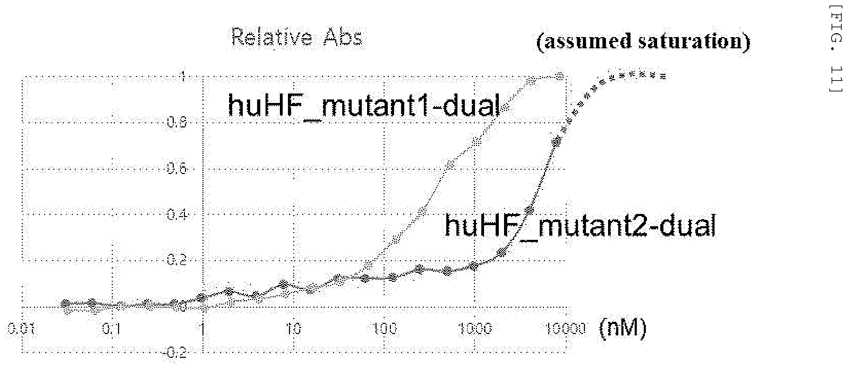

FIG. 11 illustrates results of evaluation/comparison of the binding force of huHF_mutant1-dual and huHF_mutant2-dual to hTfR. Referring to this drawing, in the case of huHF_mutant2-dual, even when the concentration of the protein was increased to 10000 nM, it was not saturated, thereby demonstrating that the binding force to hTfR was further reduced. When the expected saturation point is selected and the binding force Kd value is calculated, it becomes about 4400 nM.

## 8. Assessment of binding ability between NK cells and cancer cells according to huHF_mutant1-dual treatment

A549, a human lung cancer cell line, was labeled with CFSE as a fluorescent material. $10^6$ cells were reacted

with CFSE at a concentration of 5 $\mu$M in an incubator at 37 °C for 20 minutes, washed with PBS, and cultured for one day by diluting $10^5$ cells in 2 ml culture media on a 2 ml cell plate.

Thereafter, $10^5$ NK cells were reacted with the A549 cell line stained with CFSE.

At this time, 10 $\mu$l of PBS was used as a control, while 10 $\mu$l of 3 $\mu$M huHF_mutant1-dual (dICB, dual immune checkpoint blocker) labeled with Cy5.5 fluorescent dye was added as an experimental group.

After incubation for 10 minutes in an incubator, NK cells were labeled using an NK 1.1 antibody-PE antibody.

Samples were fixed with 4% formaldehyde, and fluorescence images were identified by fluorescence microscopy.

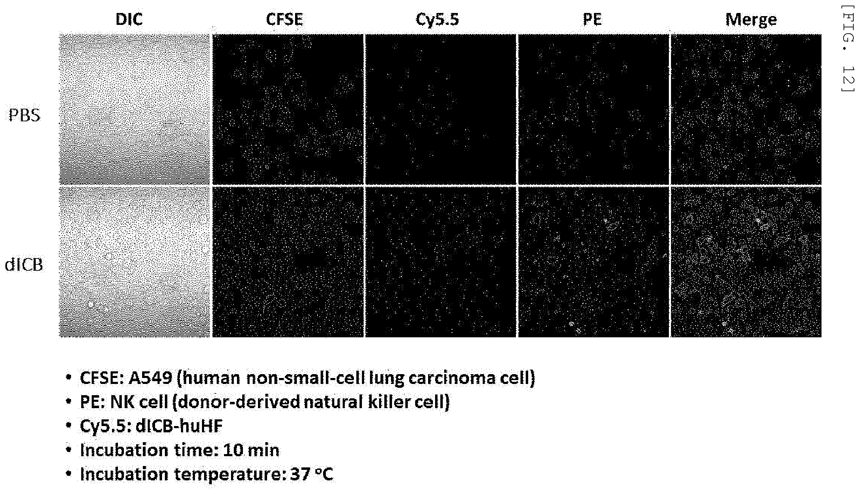

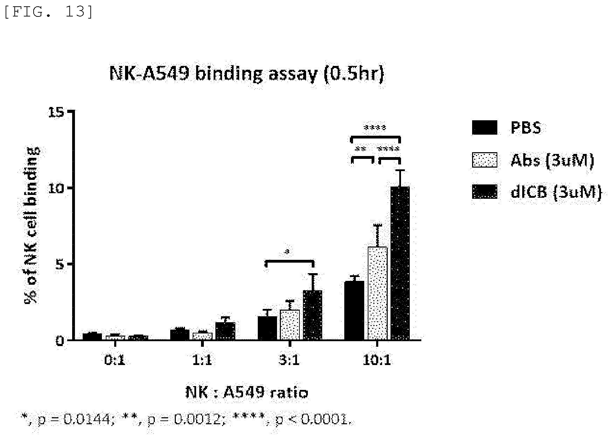

It was confirmed that more NK cells were adhered to A549 cells in the fluorescence image of the experimental group (huHF_mutant1-dual) compared to the control (PBS) (FIGS. 12 and 13).

### 9. Assessment of binding ability between NK cells and cancer cells and cancer cell killing ability of NK cells according to huHF_mutant1-dual treatment

A549, a human lung cancer cell line, was labeled with CFSE as a fluorescent material. $10^6$ cells were reacted with CFSE at a concentration of 5 $\mu$M in an incubator at 37 °C for 20 minutes, washed with PBS, and cultured for one day by diluting $10^4$ cells in 200 $\mu$l culture media on a 96 well plate.

Thereafter, $10^4$ to $10^5$ NK cells labeled with DAPI were reacted with the A549 cell line stained with CFSE depending on the conditions.

At this time, 10 $\mu$l of PBS, 10 $\mu$l of 3 $\mu$M Abs (Tecentriq+mAb-mTIGIT (Bio X Cell, #BE0274) coadministration group) (each 3 $\mu$M, total 6 $\mu$M), and 10 $\mu$l of 3 $\mu$M huHF_mutant1-dual were added.

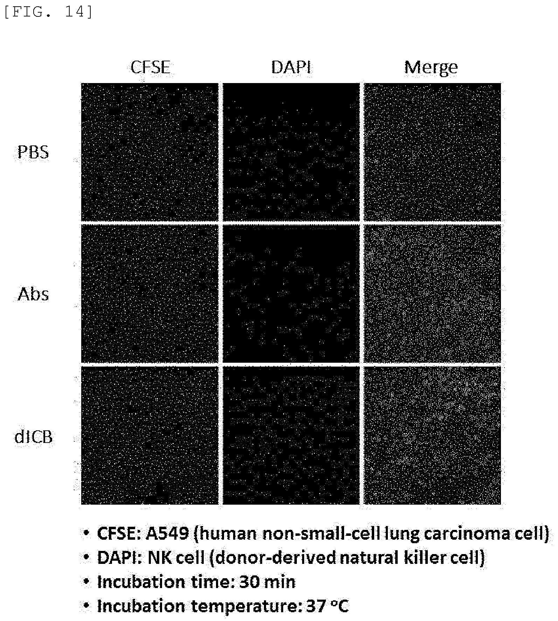

For binding measurement, the reactant solution was reacted in an incubator for 30 minutes, and then, a ratio of cells simultaneously exhibiting CFSE fluorescence and DAPI fluorescence was analyzed with FACS equipment (FIG. 14). Referring to this, it could be confirmed that the binding ability of NK cells to A549 cells is increased during huHF_mutant1-dual treatment.

Fluorescence microscopy was used to confirm the fluorescence image (FIG. 14). NK cells were labeled with the NK 1.1 antibody-PE antibody.

For toxicity measurement, the reactant solution was reacted in an incubator for 48 hours, and then, toxic ability according to the sample was verified with FACS equipment. A degree of cell death was analyzed with 7-AAD.

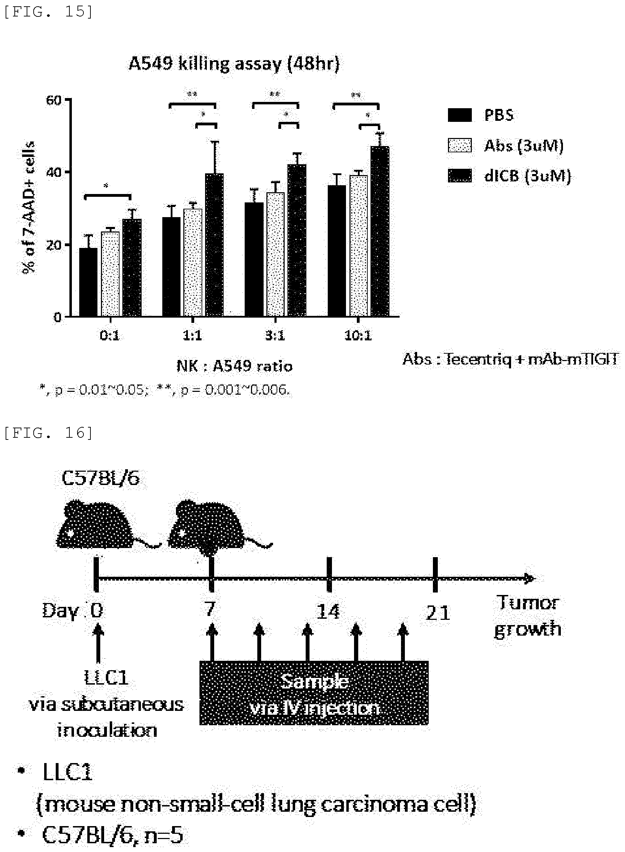

By analyzing a ratio of cells simultaneously exhibiting CFSE fluorescence and 7-AAD fluorescence, a ratio of NK cells to cancer cells and the toxic ability of NK cells according to the sample were analyzed (FIG. 15). Referring to this, it could be confirmed that A549 cell killing ability of NK cells is increased during huHF _mutant1-dual treatment.

### 10. Assessment of cancer cell growth inhibitory ability

For each of the following sample groups (PBS, Tecentriq, Tecentriq+mAb-mTIGIT and huHF_mutant-dual), the efficacy of inhibiting normal cancer cells against LLC1, a mouse lung cancer cell line, was verified by 5 mice each. Tecentriq is a monoclonal antibody that binds to PD-1 or PD-L1 used for the treatment of lung cancer, etc., while mAb-mTIGIT is a monoclonal antibody that binds to murine TIGIT.

A tumor model was formed by subcutaneously transplanting LLC1 into C57BL/6 by 5 x $10^5$ each.

After 7 days, the tumor size was measured and classified for each individual, and the same size was adjusted for each group.

From the 7th day, the sample was intravenously injected through the caudal vein every 3 days, and a tumor size was measured for each individual to compare the tumor growth inhibition efficacy (FIG. 16).

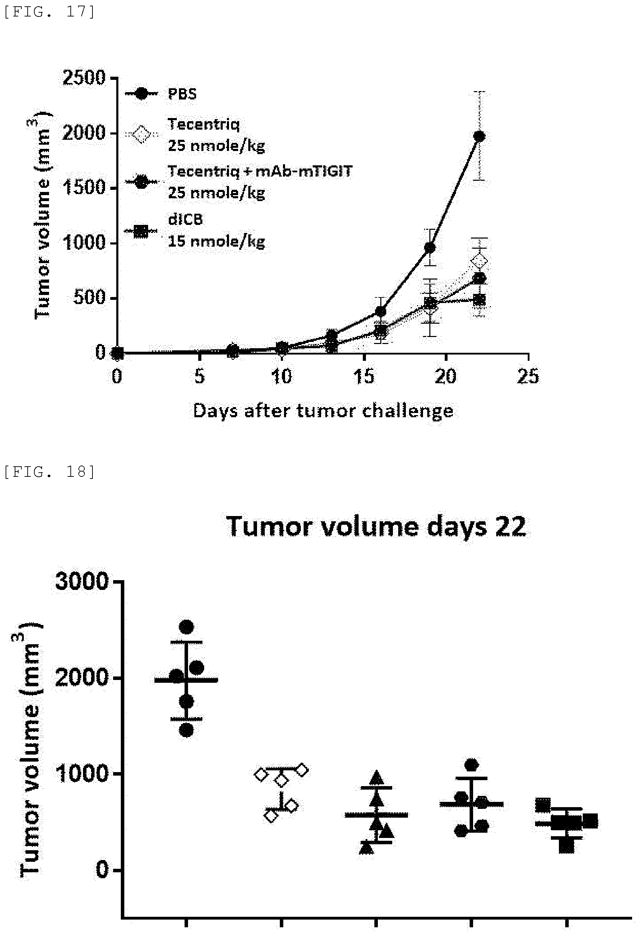

FIG. 17 shows the tumor size by date in each experimental group, in particular, FIG. 18 shows the tumor size on day 22 in each experimental group. Referring to these drawings, in the case of huHF_mutant-dual, it could be confirmed that effects are excellent because the tumor size is the smallest despite treatment with a smaller dose.

### 11. Cell avidity assessment

The avidity of huHF_mutant-dual and mut-huHF against human lung cancer cell line A549 was analyzed.

First, target cells (immune cells, cancer cells) were cultured and dispensed.

(1 x $10^5$ pieces/10 $\mu$l, 1 FACS column (5 ml polystyrene round-bottom tube, Falcon (352052))).

Then, the sample (drug, antibody (anti-mouse PD-L1 antibody (BE0101), anti-mouse TIGIT antibody (BE0274), anti-human PD-L1 (BE0285))) was treated with 100 $\mu$l/FACS column, and the control was treated with 100 ul of FACS buffer (0.1% BSA + 0.01% sodium azide in PBS) (3hr, room temperature).

After putting 1 ml of each FACS buffer into the FACS column, centrifugation was performed at 1,700 rpm for 5 minutes, and the process of removing the supernatant was repeated 3 times.

Thereafter, fluorescence antibody (anti-his tag antibody-PE (SC-8036PE), anti-IgG antibody-PE (PE goat F(ab')2 anti-rat (IgG) secondary antibody (ab7010), PE F(ab')2-goat anti-mouse IgG (H+L) secondary antibody (12-4010-82))) was used for treatment (2 hr, room temperature).

After putting 1 ml of each FACS buffer into the FACS column, centrifugation was performed at 1,700 rpm for 5 minutes, and the process of removing the supernatant was repeated 3 times.

Then, after putting 100 $\mu$l of FACS buffer in each sample FACS column, it was treated with 2 $\mu$l of 7-AAD (BD Pharmigen (559925)) for 5 minutes.

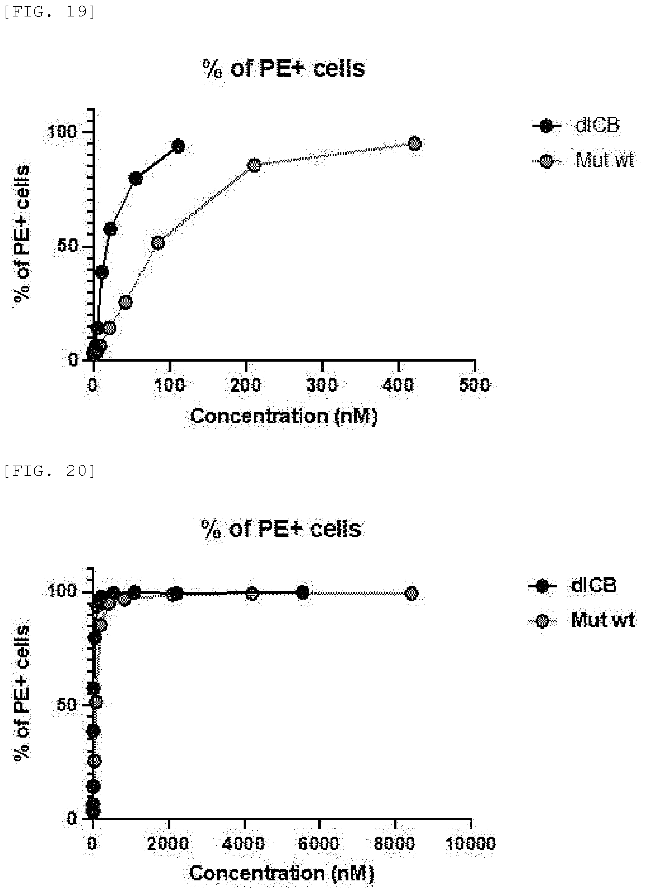

Analysis was performed by means of BD Biosciences and FACS equipment using the ratio of target cells showing PE fluorescence among live cells in each FACS column (FIGS. 19-21).

Through this, it was verified that huHF_mutant-dual had a greater number of samples bound per cell and superior number of bound cells.

## 12. Assessment of tumor growth inhibitory ability

For each of the following sample groups (PBS, Ab-1, huHF_mutant-dual and huHF_mutant-dual+Vac), the efficacy of inhibiting normal cancer cells was verified against LLC1, a mouse lung cancer cell line, by 5 mice each. Ab-1 used herein was mouse anti-PD-L1 antibody (Bio X Cell, #BE0101), and LLC-1 neo-antigen vaccine (SEQ ID NO: 10) was used as the vaccine (Vac).

A tumor model was formed by subcutaneously transplanting LLC1 into C57BL/6 by 5 x 10$^5$.

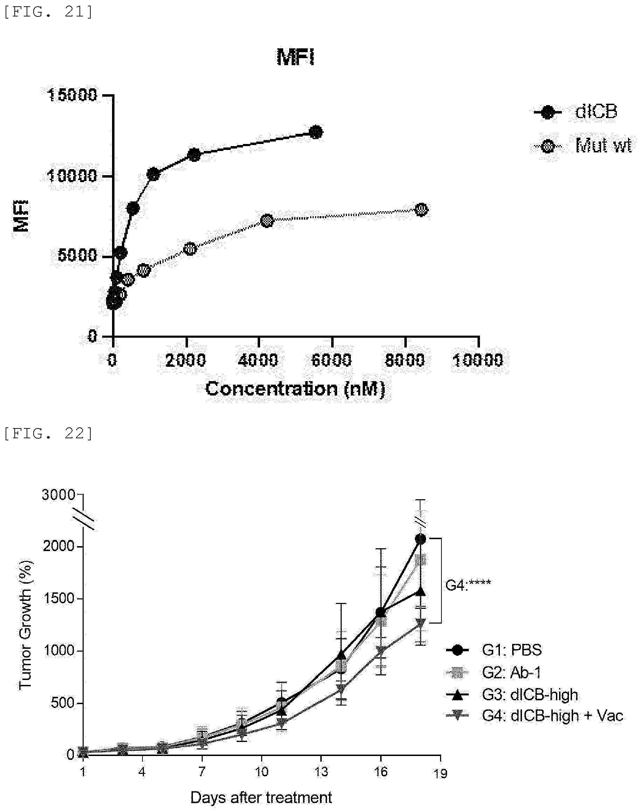

From the 7th day, the sample was intravenously injected through the caudal vein every 3 days (in the case of the G2 group, injected every 6 days), and the tumor size was measured for each individual to compare the tumor growth inhibition efficacy (FIG. 22).

Ab-1 was injected at a concentration of 30 nmol/kg, huHF_mutant-dual was 30 nmol/kg, and Vac was 10 nmol/kg.

Referring to this result, it could be confirmed that the huHF_mutant-dual treatment group has excellent tumor growth inhibitory ability, and exhibits more excellent effects when using in combination with a vaccine.

## 13. Ferritin protein fused with foreign peptide inside helix

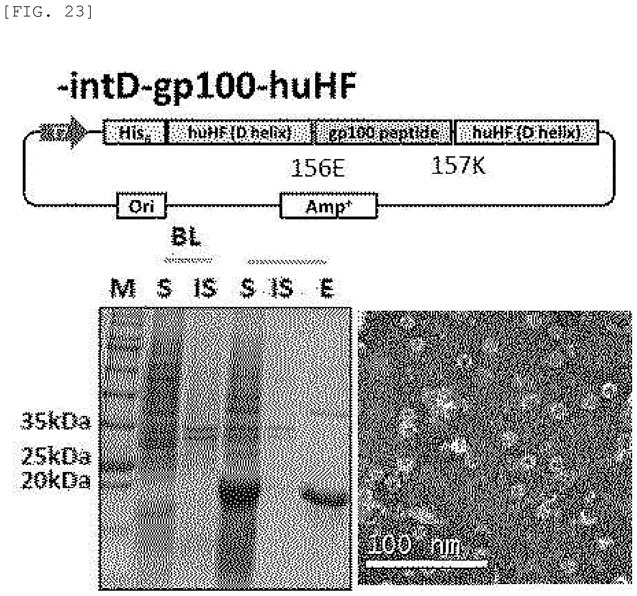

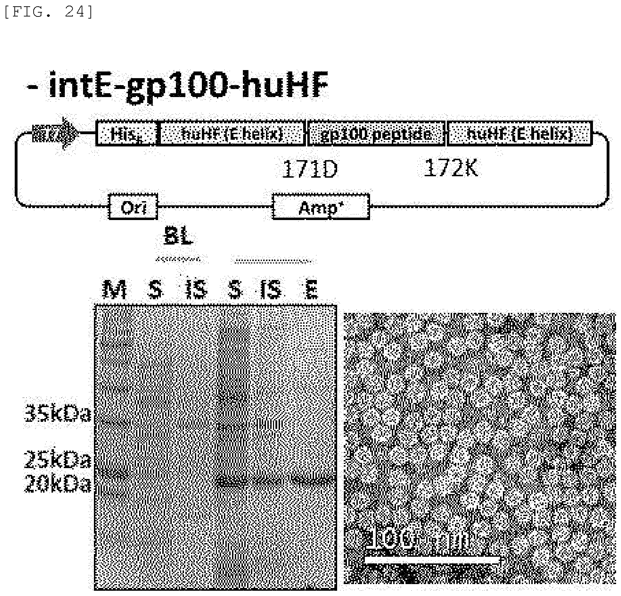

An expression vector for protein production was constructed in the same manner as in Example 1, except that the vector schematic diagrams in FIGS. 23 and 24 were used. Then, proteins were biosynthesized and purified by the methods described in Examples 2 and 3.

Referring to this result, it could be seen that the huHF structure is smoothly formed even when a foreign peptide is inserted (fused) in the middle of the D helix and in the middle of the E helix, as well as the loop between the A-E helix of huHF.

This is considered to be due to the fact that the middle of the helix, particularly the D helix and the E helix, has relatively low importance in terms of retaining the function/structure of ferritin, and the size of the fused peptide is not large.

## 14. Determination of the ratio of water-soluble fractions of proteins

BL21 (DE3) competent cells were transformed with various pT7-7-based expression vectors. A single colony was inoculated in LB liquid medium (50 mL) to which 100 mg/L of ampicillin was added, and cultured at 37 °C and 130 rpm in a shaking incubator. When the turbidity/optical density at 600 nm reached 0.5, the expression of a target protein was induced through administration of 1 mM IPTG. After 12-16 hours of incubation at 20°C, the cells in the culture medium were spun-down through centrifugation (13,000 rpm, 10 minutes), and cell pellets were collected and resuspended in 10 mM Tris-Hcl (pH 7.4) buffer. The resuspended cells were ruptured using a Branson Sonifier (Branson Ultrasonics Corp., Danbury, CT). After sonication, the supernatant containing soluble protein and aggregates containing insoluble protein were separated by centrifugation (13,000 rpm, 10 min). The separated soluble and insoluble protein fractions were analyzed for solubility by SDS-PAGE analysis. That is, the target protein bands stained with Coomassie were scanned with a densitometer (Duoscan T1200, Bio-Rad, Hercules, CA), followed by quantifying the ratio of soluble fractions. Specifically, after setting a band thickness and background value with 'Quantity One' program 'Volume Rect. Tool' using the scanned SDS-PAGE gel image, the sum of soluble and insoluble protein fractions was set to 100% using 'Volume Analysis Report', followed by quantifying the solubility.

Results thereof are shown in Table 6 below. Referring to this table, it could be seen that the water solubility ratio is high even when the foreign peptide is fused inside the D helix or inside the E helix.

[TABLE 6]

| Protein NP | Solubility (%) |
|---|---|
| intD-gp100-huHF | 48.21 |
| intE-gp100-huHF | 73.00 |

**Claims**

1. A pharmaceutical composition for preventing or treating lung cancer, comprising a ferritin protein in which a molecule capable of binding to an immune checkpoint molecule is fused to an outer surface of the protein.

2. The pharmaceutical composition according to claim 1, wherein the protein is a spherical protein formed by self-assembly of 24 ferritin monomers to which the molecule capable of binding to an immune checkpoint molecule is fused.

3. The pharmaceutical composition according to claim 1, wherein the molecule capable of binding to an immune checkpoint molecule is fused to at least one between adjacent α-helices of the ferritin monomer.

4. The pharmaceutical composition according to claim 1, wherein the molecule capable of binding to an immune checkpoint molecule is fused to N-terminus or C-terminus of the ferritin monomer.

5. The pharmaceutical composition according to claim 1, wherein the molecule capable of binding to an immune checkpoint molecule is fused to A-B loop, B-C loop, C-D loop or D-E loop of the ferritin monomer.

6. The pharmaceutical composition according to claim 1, wherein the molecule capable of binding to an immune checkpoint molecule is fused between N-terminus and A helix or between E helix and C-terminus of the ferritin monomer.

7. The pharmaceutical composition according to claim 1, wherein the molecule capable of binding to an immune checkpoint molecule is fused inside α-helix in the ferritin monomer.

8. The pharmaceutical composition according to claim 1, wherein the molecule capable of binding to an immune checkpoint molecule is fused inside A helix, B helix, C helix, D helix or E helix of the ferritin monomer.

9. The pharmaceutical composition according to claim 1, wherein the protein is mutated so that a binding force to a human transferrin receptor is reduced.

10. The pharmaceutical composition according to claim 1, wherein at least one among ferritin monomers constituting the protein is **characterized in that** amino acid No. 15, 16, 23, 82, 84, 117, 120 or 124 in the sequence of SEQ ID NO: 1 is substituted with alanine, glycine, valine or leucine.

11. The pharmaceutical composition according to claim 1, wherein the binding force (K) to the transferrin receptor satisfies Equation 1 below:

$$[Equation\ 1]$$

$$K \geq 10\ nM$$

(in Equation 1, K is [P][T]/[PT], wherein [P] represents a concentration of ferritin protein in an equilibrium state of a binding reaction between the ferritin protein and the transferrin receptor, [T] represents a concentration of the transferrin receptor in the equilibrium state, and [PT] represents a concentration of a complex of the ferritin protein and the transferrin receptor in the equilibrium state).

12. The pharmaceutical composition according to claim 10, wherein the transferrin receptor is a human transferrin receptor.

**13.** The pharmaceutical composition according to claim 1, wherein the immune checkpoint molecule is any one selected from the group consisting of Her-2/neu, VISTA, 4-1BBL, Galectin-9, Adenosine A2a receptor, CD80, CD86, ICOS, ICOSL, BTLA, OX-40L, CD155, BCL2, MYC, PP2A, BRD1, BRD2, BRD3, BRD4, BRDT, CBP, E2F1, MDM2, MDMX, PPP2CA, PPM1D, STAT3, IDH1, PD1, CTLA4, PD-L1, PD-L2, LAG3, TIM3, TIGIT, BTLA, SLAMF7, 4-1BB, OX-40, ICOS, GITR, ICAM-1, BAFFR, HVEM, LFA-1, LIGHT, NKG2C, SLAMF7, NKp80, LAIR1, 2B4, CD2, CD3, CD16, CD20, CD27, CD28, CD40L, CD48, CD52, EGFR family, AXL, CSF1R, DDR1, DDR2, EPH receptor family, FGFR family, VEGFR family, IGF1R, LTK, PDGFR family, RET, KIT, KRAS, NTRK1 and NTRK2.

**14.** The pharmaceutical composition according to claim 1, wherein the molecule capable of binding to an immune checkpoint molecule is a ligand or a fragment thereof, a receptor or a fragment thereof, an antibody or a fragment thereof including an antigen binding region (CDR), which have a binding force to the immune checkpoint molecule.

**15.** The pharmaceutical composition according to claim 1, wherein the ferritin is a human ferritin heavy chain.

**16.** The pharmaceutical composition according to claim 1, further comprising an anticancer agent or wherein the composition is co-administered with the anticancer agent.

[FIG. 1]

[FIG. 2]

His₆  D,K  Mut huHF (A,B)  antibody HCDR3 peptide  Mut huHF (C,D,E)  antibody HCDR2 peptide

92     93

Ori     Kanʳ

BL S IS  S  IS     Elu          TEM image

35 kDa

25 kDa
20 kDa

[FIG. 3]

[FIG. 4]

Cell type : Mouse Lung Cancer (Lewis Lung Cancer;LLC1)
Sample Fluorescence Intensity : 1400

α-mouse_PD-L1

α-mouse_TIGIT

huHF-h_smPD1_Mutant

huHF-dual_Mutant

huHF_Native

% of LLC1

No treat
α-mouse_PD-L1
α-mouse_TIGIT
huHF_Native
huHF-dual_Mutant
huHF-h_smPD1_Mutant

[FIG. 5]

[FIG. 6]

Cell type : Mouse Lung Cancer (Lewis Lung Cancer;LLC1)

α-mouse_TIGIT
huHF_Mutant-dual

Concentration (nM)

Cell fraction (%)

Cell type : Human Lung Cancer (A549)

[FIG. 7]

EP 4 335 450 A1

[FIG. 8]

[FIG. 9]

[FIG. 10]

huHF_Mutant-dual

hTfR 181.9 nM

[FIG. 11]

Relative Abs

(assumed saturation)

huHF_mutant1-dual

huHF_mutant2-dual

(nM)

[FIG. 12]

|  | DIC | CFSE | Cy5.5 | PE | Merge |
|---|---|---|---|---|---|
| PBS |  |  |  |  |  |
| dICB |  |  |  |  |  |

- CFSE: A549 (human non-small-cell lung carcinoma cell)
- PE: NK cell (donor-derived natural killer cell)
- Cy5.5: dICB-huHF
- Incubation time: 10 min
- Incubation temperature: 37 °C

[FIG. 13]

**NK-A549 binding assay (0.5hr)**

*, p = 0.0144; **, p = 0.0012; ****, p < 0.0001.

[FIG. 14]

|  | CFSE | DAPI | Merge |

- **CFSE: A549 (human non-small-cell lung carcinoma cell)**
- **DAPI: NK cell (donor-derived natural killer cell)**
- **Incubation time: 30 min**
- **Incubation temperature: 37 °C**

[FIG. 15]

**A549 killing assay (48hr)**

*, p = 0.01~0.05; **, p = 0.001~0.006.

Abs : Tecentriq + mAb-mTIGIT

[FIG. 16]

- LLC1
  (mouse non-small-cell lung carcinoma cell)
- C57BL/6, n=5

[FIG. 17]

[FIG. 18]

## Tumor volume days 22

[FIG. 19]

**% of PE+ cells**

[FIG. 20]

**% of PE+ cells**

[FIG. 21]

[FIG. 22]

[FIG. 23]

# -intD-gp100-huHF

[FIG. 24]

- intE-gp100-huHF

## INTERNATIONAL SEARCH REPORT

| | International application No. |
|---|---|
| | **PCT/KR2022/006352** |

### A. CLASSIFICATION OF SUBJECT MATTER

**A61K 38/00**(2006.01)i; **A61K 38/17**(2006.01)i; **A61K 39/00**(2006.01)i; **A61P 35/00**(2006.01)i

According to International Patent Classification (IPC) or to both national classification and IPC

### B. FIELDS SEARCHED

Minimum documentation searched (classification system followed by classification symbols)

A61K 38/00(2006.01); A61K 31/69(2006.01); A61K 38/16(2006.01); A61K 9/51(2006.01); C07K 14/47(2006.01); C07K 14/79(2006.01); C07K 19/00(2006.01); C12N 15/62(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 페리틴(ferritin), 트랜스페린 수용체(transferrin receptor), 융합(fusion), 돌연변이(mutation), 암(cancer)

### C. DOCUMENTS CONSIDERED TO BE RELEVANT

| Category* | Citation of document, with indication, where appropriate, of the relevant passages | Relevant to claim No. |
|---|---|---|
| X | KR 10-2018-0008353 A (KOREA INSTITUTE OF SCIENCE AND TECHNOLOGY) 24 January 2018 (2018-01-24)<br>See claims 1-2, 4-5 and 7-19; paragraphs [0014], [0026], [0036]-[0037] and [0063]; and figures 1a-1b. | 1-8,13-16 |
| Y | | 9-12 |
| Y | MONTEMIGLIO, L. C. et al. Cryo-EM structure of the human ferritin–transferrin receptor 1 complex. Nature Communications. 2019, vol. 10, thesis no. 1121, pp. 1-8.<br>See abstract; page 4; figure 3; and supplementary figure 4. | 9-12 |
| X | KR 10-2017-0027683 A (KYUNGPOOK NATIONAL UNIVERSITY INDUSTRY-ACADEMIC COOPERATION FOUNDATION et al.) 10 March 2017 (2017-03-10)<br>See abstract; claims 1-18; and paragraphs [0062]-[0066] and [0085]-[0103]. | 1-8,13-16 |
| Y | | 9-12 |

| ✓ Further documents are listed in the continuation of Box C. | ✓ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **18 August 2022** | **18 August 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2022/006352**

**C.     DOCUMENTS CONSIDERED TO BE RELEVANT**

| Category* | Citation of document, with indication, where appropriate, of the relevant passages | Relevant to claim No. |
|---|---|---|
| | KR 10-2017-0047120 A (KOREA UNIVERSITY RESEARCH AND BUSINESS FOUNDATION et al.) 04 May 2017 (2017-05-04) | |
| X | See claims 1-5 and 9-21. | 1-8,13-16 |
| Y | | 9-12 |
| | KR 10-2015-0088597 A (UNIST ACADEMY-INDUSTRY RESEARCH CORPORATION) 03 August 2015 (2015-08-03) | |
| X | See claims 1-20; and paragraph [0021]. | 1-8,13-16 |
| Y | | 9-12 |

<table>
<tr><td>**INTERNATIONAL SEARCH REPORT**</td><td>International application No.<br>**PCT/KR2022/006352**</td></tr>
</table>

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (July 2019)

**INTERNATIONAL SEARCH REPORT**
Information on patent family members

International application No.

**PCT/KR2022/006352**

| Patent document cited in search report | | | Publication date (day/month/year) | Patent family member(s) | | | Publication date (day/month/year) |
|---|---|---|---|---|---|---|---|
| KR | 10-2018-0008353 | A | 24 January 2018 | CA | 3040440 | A1 | 18 January 2018 |
| | | | | CN | 109803642 | A | 24 May 2019 |
| | | | | EP | 3501509 | A1 | 26 June 2019 |
| | | | | EP | 3501509 | A4 | 08 July 2020 |
| | | | | KR | 10-1953617 | B1 | 23 May 2019 |
| | | | | US | 10905774 | B2 | 02 February 2021 |
| | | | | US | 2019-0216947 | A1 | 18 July 2019 |
| | | | | WO | 2018-012952 | A1 | 18 January 2018 |
| KR | 10-2017-0027683 | A | 10 March 2017 | KR | 10-1888675 | B1 | 14 August 2018 |
| | | | | US | 10781238 | B2 | 22 September 2020 |
| | | | | US | 2018-0291072 | A1 | 11 October 2018 |
| | | | | WO | 2017-039382 | A1 | 09 March 2017 |
| KR | 10-2017-0047120 | A | 04 May 2017 | KR | 10-2389169 | B1 | 21 April 2022 |
| | | | | US | 10206987 | B2 | 19 February 2019 |
| | | | | US | 2017-0112910 | A1 | 27 April 2017 |
| KR | 10-2015-0088597 | A | 03 August 2015 | KR | 10-1630858 | B1 | 15 June 2016 |

Form PCT/ISA/210 (patent family annex) (July 2019)